Europäisches Patentamt

European Patent Office

Office européen des brevets

(19)

(11) Veröffentlichungsnummer: **0 359 984**
A1

# (12) EUROPÄISCHE · PATENTANMELDUNG

(21) Anmeldenummer: 89114914.8

(51) Int. Cl.5: **B60H 3/00** , **A61L 9/12**

(22) Anmeldetag: **11.08.89**

(30) Priorität: **22.08.88 IT 5336688 U**

(43) Veröffentlichungstag der Anmeldung:
**28.03.90 Patentblatt 90/13**

(84) Benannte Vertragsstaaten:
**AT BE CH DE ES FR GB GR LI LU NL SE**

(71) Anmelder: **D'Amelio, Antonio**
**via Principessa Felicita di Savoia, 15**
**I-10131 Torino(IT)**

(72) Erfinder: **D'Amelio, Antonio**
**via Principessa Felicita di Savoia, 15**
**I-10131 Torino(IT)**

(74) Vertreter: **Aprà, Andrea, Dipl.-Ing. Dr. jur. et al**
**Via Cernaia 27**
**I-10121 Torino(IT)**

(54) Parfüm-Spender für eine Zwangsbelüftung, insbesondere in Kraftfahrzeugen.

(57) Parfüm-Spender für eine Zwangsbelüftungsanlage insbesondere in Kraftfahrzeugen, erfindungsgemäss umfassend einen Behälter (11) für eine Parfüm-Flüssigkeit und eine Pumpe (12), die mit einem Ausgang des Behälters für die Parfüm-Flüssigkeit abgedichtet hydraulisch verbunden ist und diese Flüssigkeit unter Druck einer Zerstäuberdüse (13) zuführt, durch die die zerstäubte Parfüm-Flüssigkeit in einen Luftkanal (C) einer Zwangsbelüftungsanlage (I) nebelförmig eindüsbar ist, sodass durch Betätigen der erwähnten Pumpe (12) die durch die Düse (13) zerstäubte Parfüm-Flüssigkeit nebelförmig in den Luftstrom der Zwangsbelüftungsanlage eingedüst und über die genannte Anlage in den zu belüftenden Raum eingeführt wird.

EP 0 359 984 A1

## Parfüm-Spender für eine Zwangsbelüftung, insbesondere in Kraftfahrzeugen

Vorliegende Erfindung betrifft einen Parfüm-Spender für Zwangsbelüftungsanlagen, insbesondere in Kraftfahrzeugen. Bekanntlich bilden sich in geschlossenen Räumen, in denen die Lufterneuerung durch Zwangsbelüftung besorgt wird, andauernde üble Gerüche, wie z.B. Tabakgeruch, Schweissgeruch u.dgl. Dieser unerwünschte Umstand wirkt sich z.B. im Fahrgastraum von Kraftfahrzeugen oder in klein bemessenen Wohnungs- oder Arbeitsräumen, die im allgemeinen geschlossen bleiben und mit mechanischer Belüftung versehen sind, in besonders starkem Masse lästig aus.

Der Erfindung liegt die Aufgabe zugrunde, einen Parfüm-Spender für Zwangsbelüftungsanlagen insbesondere in Kraftfahrzeugen als wirksame Abhilfe für den erwähnten nachteiligen Umstand vorzusehen.

Weiterhin soll der Parfüm-Spender einfach im Aufbau, kostengünstig in der Herstellung und zuverlässlich im Betrieb sowie einbau- und wartungsfreundlich sein.

Mit Hinsicht auf diese Aufgabenstellung sieht die Erfindung einen Parfüm-Spender für Zwangsbelüftungsanlagen insbesondere in Kraftfahrzeugen vor, der gekennzeichnet ist durch einen Behälter für eine Parfüm-Flüssigkeit und durch eine pumpe, die mit einem Ausgang des Behälters für die Parfüm-Flüssigkeit abgedichtet hydraulisch verbunden ist und diese Flüssigkeit unter Druck einer Zerstäuberdüse zuführt, durch die die zerstäubte Parfüm-Flüssigkeit in einen Luftkanal einer Zwangsbelüftungsanlage nebelförmig eindüsbar ist, sodass durch Betätigen der erwähnten Pumpe die durch die Düse zerstäubte Parfüm-Flüssigkeit nebelförmig in den Luftstrom der Zwangsbelüftungsanlage eingedüst und über die genannte Anlage in den zu belüftenden Raum eingeführt wird.

Die erwähnte Pumpe ist mit Vorteil eine Elektropumpe, die mit dem vorhandenen elektrischen Stromkreis unter Zwischenschaltung eines elektrischen Betätigungsschalters verbunden ist.

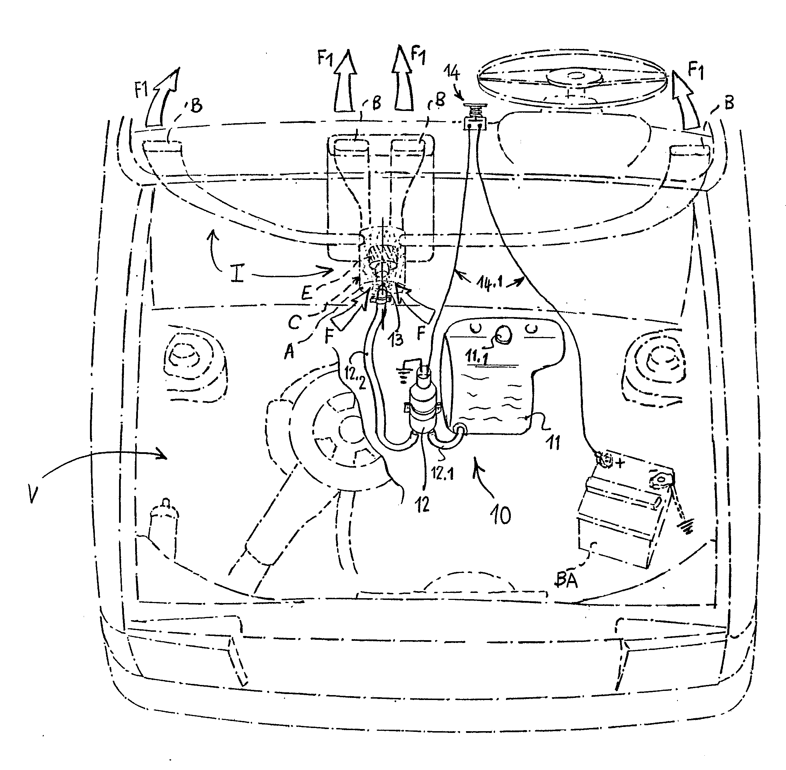

Die Erfindung ist nachstehend an einem bevorzugten Ausführungsbeispiel anhand der Zeichnung näher beschrieben; dabei zeigt die einzige Figur der Zeichnung einen erfindungsgemässen Parfüm-Spender, der in einem Kraftfahrzeug in Verbindung mit der Zwangsbelüftungsanlage für den Fahrgastraum des Fahrzeugs installiert ist.

In der Zeichnung ist mit V der Motorraum eines Kraftfahrzeuges bei geöffneter Mottorraumhaube bezeichnet. Mit I ist die Zwangsbelüftungsanlage für den Fahrgastraum des Kraftfahrzeuges mit einem Elektrolüfter E in einem zur Zwangsbelüftung dieses Raumes Luft unter Druck fördernden Kanal C bezeichnet. A bezeichnet die Lufthaube für den Kanal C und B die vom Kanal C ausgehenden Belüftungsdüsen für den Innenraum des Kraftfahrzeugs. Mit BA ist noch die Batterie zur Versorgung des Kraftfahrzeuges mit elektrischem Strom bezeichnet. Diese aufgeführten Teile sind zur klareren Darstellung strichpunktiert gezeichnet.

Der erfindungsgemässe Parfüm-Spender ist in seiner Gesamtheit mit 10 bezeichnet.

Per Parfüm-Spender 10 umfasst gemäss dem dargestellten Ausführungsbeispiel einen für die Parfüm-Flüssigkeit bestimmten Behälter 11, der im Motorraum V angeordnet und über einen Einfüllstutzen mit einem abnehmbaren Behälterverschluss 11.1 mit der gewünschten Parfüm-Flüssigkeit füllbar ist. Eine im Motorraum V feststehend eingebaute Elektropumpe 12 ist mit einem Ausgang des Behälters 11 über eine Saugleitung 12.1 dicht verbunden. Die Elektropumpe 12 versorgt über eine Druckleitung 12.2 eine Zerstäuberdüse 13 mit der aus dem Behälter 11 gesaugten Parfum-Flüssigkeit. Die Zerstäuberdüse 13 ist im Motorraum V im Bereich der Belüftungshaube A feststehend angeordnet und düst die Parfüm-Flüssigkeit nebelförmig verteilt in den Luftstrom im Luftkanal C der Belüftungsanlage I ein. Die Elektropumpe 12 ist mit dem Stromversorgungskreis des Kraftfahrzeugs (in der Zeichnung durch eine von der Batterie BA ausgehende elektrische Leitung 14.1 zeichnerisch angedeutet) unter Zwischenschaltung eines Druckknopfschalters 14 verbunden. Der Druckknopf- bzw. Betätigungsschalter 14 hält den von der Elektropumpe 12 abgezweigten Stromkreis (elektrische Leitung 14.1) normalerweise offen. Durch Betätigung des Schalters 14 wird dieser Stromkreis geschlossen und dadurch die Elektropumpe 12 in Betrieb gesetzt. Die Parfüm-Flüssigkeit wird daher aus dem Behälter 11 gesaugt, unter Druck der Zerstäuberdüse 13 zugeführt und durch diese zerstäubt sowie über die Belüftungshaube A in nebelförmiger Verteilung (durch die dichte Punktierung in der Zeichnung angedeutet) in den Kanal C eingedüst (Pfeile F in der Zeichnung). Die solchermassen parfümierte Luft wird dann über die an den Kanal C der Zwangsbelüftungsanlage I angeschlossenen Belüftungsdüsen B entsprechend verteilt in den Fahrgastraum des Kraftfahrzeugs (Pfeile F1 in der Zeichnung) eingeführt. Dadurch wird der Innenraum des Kraftfahrzeugs gleichförmig und einheitlich unter Beseitigung der üblen bzw. lästigen Gerüche parfümiert. Wird dann der elektrische Schalter 14 losgelassen, so tritt die Elektropumpe 12 ausser Betrieb und die Eindüsung der Parfüm-Flüssigkeit durch die Zerstäuberdüse 13 in die Zwangsbelüftungsanlage I wird unterbrochen.

Es versteht sich, dass praktisch zahlreiche Änderungen gegenüber dem beschriebenen und dargestellten Ausführungsbeispiel zulässig sind.

So kann z.B. statt einer Elektropumpe eine von Hand betätigbare Kleinpumpe, wie sie beispielsweise auch zum Bespritzen der Windschutzscheibe von Kraftfahrzeugen mit Waschflüssigkeit dient, benutzt werden. Ausserdem kann die Zerstäuberdüse wahlweise hinter oder vor dem Elektrolüfter der Zwangsbelüftungsanlage oder an beliebiger Stelle im Luftkanal dieser Anlage angeordnet werden.

Der Parfüm-Spender ist hier zwar in Verbindung mit einer Zwangsbelüftungsanlage für Kraftfahrzeuge beschrieben und dargestellt worden, doch kann dieser ohne weiters auch in Verbindung mit anderen Zwangsbelüftungsanlagen (z.B.) Klima-Anlagen) für Wohnungs- oder Arbeitsräume installiert werden.

In Kraftfahrzeugen kann der Parfüm-Spender auch in einem vom Motorraum verschiedenen Raum angeordnet sein, ebenso wie auch der zugehörige Behälter und die zugehörige Pumpe auch an untereinander verschiedenen Stellen installiert werden können.

## Ansprüche

1. Parfüm-Spender für eine Zwangsbelüftungsanlage, insbesondere in Kraftfahrzeugen, gekennzeichnet durch einen Behälter (11) für eine Parfüm-Flüssigkeit und durch eine Pumpe (12), die mit einem Ausgang des Behälters für die Parfüm-Flüssigkeit abgedichtet hydraulisch verbunden ist und diese Flüssigkeit unter Druck einer Zerstäuberdüse (13) zuführt, durch die die zerstäubte Parfüm-Flüssigkeit in einen Luftkanal (C) einer Zwangsbelüftungsanlage (I) nebelförmig eindüsbar ist, sodass durch Betätigen der erwähnten Pumpe (12) die durch die Düse (13) zerstäubte Parfüm-Flüssigkeit nebelförmig in den Luftstrom der Zwangsbelüftungsanlage eingedüst und über die genannte Anlage in den zu belüftenden Raum eingeführt wird.

2. Parfüm-Spender nach Anspruch 1, dadurch gekennzeichnet, dass die erwähnte Pumpe (12) eine Elektropumpe ist, die mit einem Stromversorgungskreis unter Zwischenschaltung eines elektrischen Betätigungsschalters (14) elektrisch verbunden ist.

3. Parfüm-Spender nach Anspruch 2, gekennzeichnet durch einen Behälter (11) für eine Parfüm-Flüssigkeit, der in einem Raum (V) eines Kraftfahrzeugs angeordnet und über einen Einfüllstutzen mit abnehmbarem Behälterverschluss (11.1) mit der gewünschten Parfüm-Flüssigkeit füllbar ist, und durch eine Elektropumpe (12), die mit einem Ausgang des Behälters (11) über eine Saugleitung (12.1) abgedichtet hydraulisch verbunden ist und über eine Druckleitung (12.2) die aus dem Behälter (11) gesaugte Parfüm-Flüssigkeit einer Zerstäuberdüse (13) unter Druck zuführt, wobei diese Düse feststehend angeordnet und dazu bestimmt ist, die nebelförmig zerstäubte Parfum-Flüssigkeit in den Luftstrom in einem Luftkanal (C) einer Zwangsbelüftungsanlage (I) für den Innenraum des Kraftfahrzeugs einzudüsen, während die erwähnte Elektropumpe (12) unter Zwischenschaltung eines elektrischen Betätigungsschalters (14) mit dem Stromversorgundkreis des Kraftfahrzeugs (über eine elektrische Leitung 14.1) elektrisch verbunden ist, und wobei der Betätigungsschalter normalerweise den von der Elektropumpe (12) abgezweigten elektrischen Stromkreis (elektrische Leitung 14.1) geöffnet hält, während durch Betätigen des elektrischen Schalters (14) dieser Stromkreis geschlossen gehalten und die Elektropumpe (12) in Betrieb gesetzt wird.

4. Parfüm-Spender nach Anspruch 1, dadurch gekennzeichnet, dass die erwähnte Pumpe eine von Hand betätigbare Kleinpumpe ist.

EP 0 359 984 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | US-A-4 805 520  (C. FREEDMAN) <br> * Insgesamt * <br> --- | 1-3 | B 60 H    3/00 <br> A 61 L    9/12 |
| Y | US-A-3 259 050  (J. GRIMM) <br> * Insgesamt * <br> --- | 1,2,4 | |
| Y <br><br> A | FR-A-2 609 669  (J. LIAUTAUD) <br> * Insgesamt * <br><br> --- | 1,2,4 <br><br> 3 | |
| A | DE-A-2 832 416  (W. CLAUSS) <br> * Insgesamt * <br> --- | 1-3 | |
| A | PATENT ABSTRACTS OF JAPAN, Band 8, Nr. 240 (M-336)[1677], 6. November 1984; & JP-A-59 120 514 (NIPPON DENSO K.K.) 12-07-1984 <br> ----- | 1-3 | |

| | |
|---|---|
| | RECHERCHIERTE SACHGEBIETE (Int. Cl.5) <br><br> B 60 H <br> A 61 L |

Der vorliegende Recherchenbericht wurde für alle Patentansprüche erstellt

| Recherchenort | Abschlußdatum der Recherche | Prüfer |
|---|---|---|
| DEN HAAG | 01-12-1989 | CZAJKOWSKI A.R. |

EPO FORM 1503 03.82 (P0403)